# EUROPEAN PATENT APPLICATION

(11) **EP 4 768 497 A1**
(43) Date of publication of application: **01.07.2026**
(21) Application number: 24855886.8
(22) Date of filing: 23.08.2024
(51) Int. Cl.: C07K 1/06, C07K 1/16, C07K 1/34, A61P 35/00

(54) **PREPARATION METHOD FOR LINKER-DRUG**

(30) Priority: 23.08.2023 CN 202311077479; 19.08.2024 CN 202411139357
(71) Applicant: SystImmune, Inc., Redmond WA 98052 (US)
(72) Inventor: ZHU, Yi, Chengdu, Sichuan 611130 (CN); WAN, Weili, Chengdu, Sichuan 611130 (CN); ZHANG, Yiying, Chengdu, Sichuan 611130 (CN); ZHU, Guili, Chengdu, Sichuan 611130 (CN); XIAO, Jie, Chengdu, Sichuan 611130 (CN)
(74) Representative: Mathys & Squire
(86) International application number: PCT/CN2024/114153
(87) International publication number: WO 2025/040165

(57) **Abstract**

The present application relates to a preparation method for a linker-drug compound. In particular, a preparation method for a target compound is provided. The method comprises reacting a compound represented by Formula a with a Lewis acid to obtain a crude product, and purifying the crude product to obtain a compound represented by Formula A.

## Description

### CROSS-REFERENCE TO RELATED APPLICATION

This application claims priority to the Chinese Patent Application No. CN 202311077479.4, filed August 23, 2023, and Chinese Patent Application No. CN 202411139357.8, filed August 19, 2024, the entire disclosures of which are incorporated herein by reference.

### TECHNICAL FIELD

The application relates to the field of pharmaceutical chemistry, and in particular to a method for preparing linker-drug compounds.

### BACKGROUND

Antibody-drug conjugates (ADCs) are therapeutic molecules having targeted killing effects. ADCs formed by conjugating small-molecule cytotoxic agents to antibodies through chemical linkers. ADCs are primarily used in the treatment of tumors and other diseases. In an ADC, the antibody component can specifically bind to proteins expressed on the surface of tumor cells, thereby enabling targeted delivery of the cytotoxic agent resulting in improved therapeutic efficacy and potential that traditional drugs may not be able to achieve. At present, multiple antibody-drug conjugates have been approved for marketing worldwide, and numerous additional candidates are undergoing clinical evaluation.

A linker-drug is a compound formed by linking a chemical linker to a small-molecule cytotoxic agent. The linker-drug serves as an intermediate in the preparation of an ADC and is an important component in the overall construction of antibody-drug conjugates.

The foregoing description of related technology is provided to facilitate an understanding of the technical field of the present application. It is not intended to be an admission that any of the information described herein constitutes prior art to the present application, nor should it be interpreted as an admission regarding the scope or content of the prior art.

### SUMMARY

The application provides, inter alia, linker-drug compounds and methods for making or preparing such linker-drug compounds.

In one aspect, the application provides a method for preparing a linker-drug compound represented by Formula A.

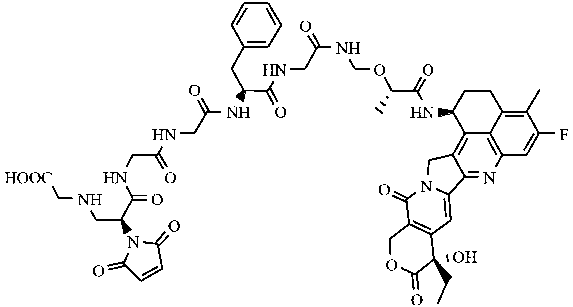

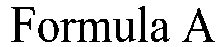

In one embodiment, a method for preparing a compound represented by Formula A, comprising: 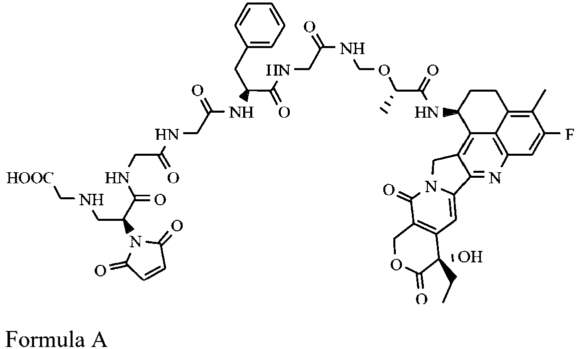 reacting the compound represented by Formula a with a Lewis acid to obtain a crude product; and purifying the crude product to obtain the compound represented by Formula A;

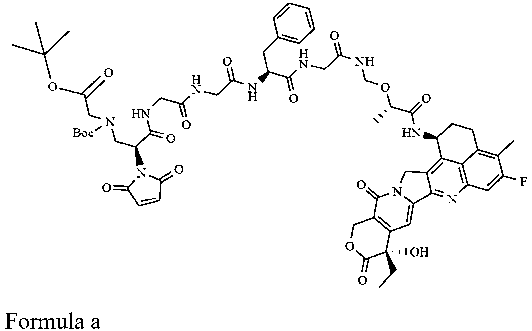

In some embodiments, the Lewis acid may be a bromide, TMSI (trimethyliodosilane), or TMSOTf (trimethylsilyl trifluoromethanesulfonate). In some embodiments, the Lewis acid is zinc bromide.

During deprotection of Compound a under a acidic condition with, for example, an acidic deprotection agent, impurities may be generated, including:
1) monodeprotection product impurity: 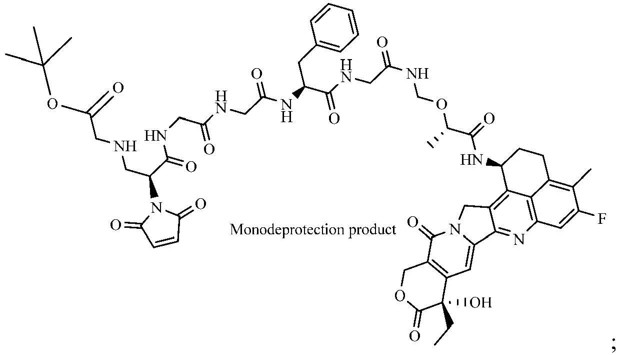
2) Compound an impurity: 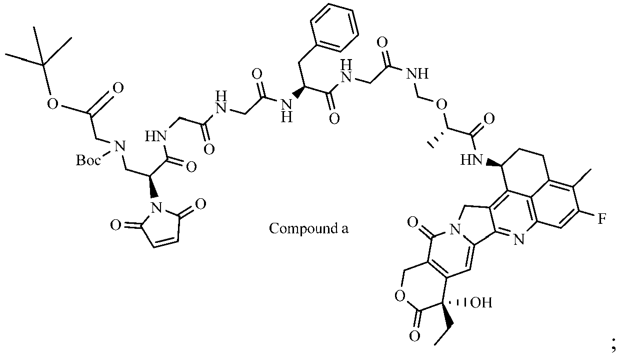
3) drug unit impurity

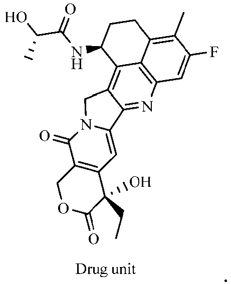

The presence of these impurities may affect the drug-to-antibody ratio (DAR) of the ultimately prepared ADC. Accordingly, in certain embodiments, the content of these impurities in the production process of the target compound may be controlled, for example, the monodeprotection product is controlled below 10%, the compound a impurity is controlled below 2%, and the drug unit impurity is controlled below 5%, to ensure the coupling efficiency between the target compound and the antibody.

Extensive experimental studies conducted by the inventors further found that, compared with non-Lewis acids (e.g., trifluoroacetic acid) as acidic deprotection agents, the use of Lewis acids (e.g., zinc bromide) as acidic deprotection agents can reduce impurity levels. In certain embodiment, the content of the drug unit impurity can be controlled to be as low as no greater than 5%, or in some embodiments to as low as no greater than 2%, therefore satisfying or even exceeding applicable quality control standards. In some embodiments, when zinc bromide is used as an acidic deprotection agent, impurity levles are significantly reduced relative to those obtained using non-Lewis acids, far exceeding applicable quality control standards.

Furthermore, during purification of the crude product obtained from the above reaction, additional impurities may be generated, including: 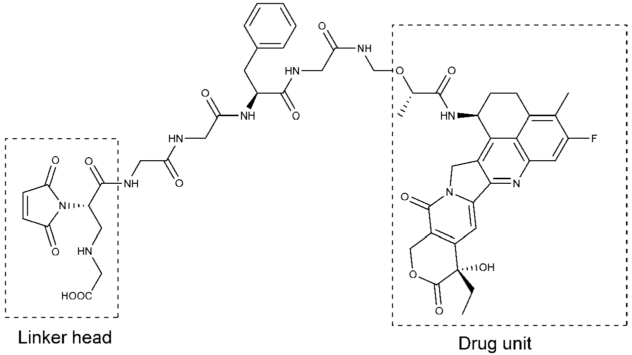
1) drug unit impurity: 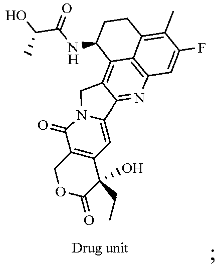
2) linker head ring-opening product impurity:

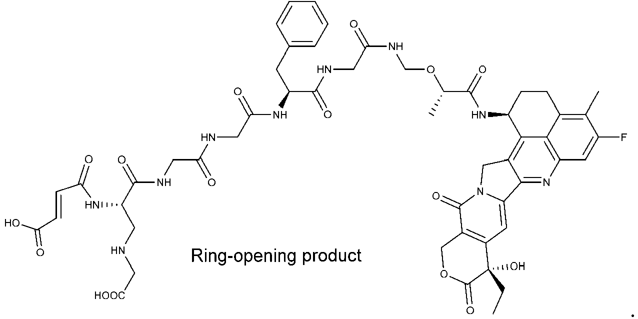

The presence of these impurities may significantly affect the drug-to-antibody ratio (DAR) of the resulting ADC. Specifically, the succinimide structure contained in the linker head of the linker-drug represented by Formula A is capable of undergoing Michael addition with a thiol group of an antibody through a double bond to form an ADC. A ring-opening product impurity may lack this reactive functionality and therefore may exhibit reduced or no coupling capability with the antibody, which can influence the DAR. In addition, formation of a drug unit impurity may result in separation of the drug moiety from the linker. The separated linker may retain the ability to couple with the antibody but would no longer deliver the interceded payload, thereby potentially affecting the DAR.

Accordingly, in certain embodiments, impurity levels are controlled during production of the target compound. For example, the ring-opening product impurity may be controlled below 10%, and the drug unit impurity must be controlled below 5%, in order to maintain effective coupling between the target compound and the antibody.

Extensive experimental research conducted by the inventors discovered that, in the above purification process, combining preparative liquid chromatography purification under certain conditions with a nanofiltration step can significantly reduce impurity level. In certain embodiments, the ring-opening product impurity is controlled below 10%, and the drug unit impurity is controlled below 5%, thereby meeting applicable product quality control standards. In certain embodiment, impurity levels may be further reduced, for example, the ring-opening product impurity may be reduced to 3% or less and the drug unit impurity to 2% or less, thereby far exceeding these standards.

In some embodiments, the method of purification comprises the following steps:
(2-1) purifying the crude product by preparative liquid chromatography to obtain a preparative solution;
(2-2) concentrating and drying the preparative solution to obtain the compound represented by Formula A;

In certain embodiments, after obtaining the preparative solution in step (2-1) and prior to step (2-2), an extraction step may be further included.

In certain embodiments, after obtaining the preparative solution in step (2-1) and prior to step (2-2), the method further include the extraction step comprising:
(2-i) extracting the preparative solution with an organic solvent, discarding the organic phase, and obtaining the aqueous phase.

In certain embodiments, in step (2-1), the mobile phase A is TFA/water, and the mobile phase B is TFA/acetonitrile when purifying using the preparative liquid chromatography. In certain embodiment, in step (2-2), the concentrating step comprises nanofiltration.

Those skilled in the art will understand that, after obtaining the aqueous phase in step (2-i), the subsequent step (2-2) may be correspondingly adjusted to comprise the step of concentrating and drying the aqueous phase to obtain the compound represented by Formula A.

In some embodiments, the method of purification comprises the following steps:
(2-1) purifying the crude product using a preparative liquid chromatography to obtain a preparative solution, when purifying using the preparative liquid chromatography, the mobile phase A is TFA/water and the mobile phase B is TFA/acetonitrile, and
(2-2) concentrating and drying the preparative solution to obtain the compound represented by Formula A by using nanofiltration.

In some embodiments, the method of purification comprises the following steps:
(2-1) purifying the crude product using a preparative liquid chromatography to obtain a preparative solution, wherein, when purifying using the preparative liquid chromatography, the mobile phase A is TFA/water and the mobile phase B is TFA/acetonitrile;
(2-i) extracting the preparative solution with an organic solvent, discarding the organic phase, and obtaining the aqueous phase; and
(2-2) concentrating and drying the aqueous phase to obtain the compound represented by Formula A by using nanofiltration;

In some embodiments, in step (2-1), when purifying using the preparative liquid chromatography, the mobile phase A is 0.2% TFA/water and the mobile phase B is 0.1% TFA/acetonitrile; in step (2-2), the concentrating is nanofiltration concentrating.

In some embodiments, in step (2-1), gradient elution is used when purifying using the preparative liquid chromatography.

In some embodiments, the gradient conditions are as follows:

| Time/min | Mobile phase A/% | Mobile phase B/% |
|---|---|---|
| 0~5 | 90~75 | 10~25 |
| 5~32 | 75~60 | 25~40 |
| 32~39 | 10~0 | 90~100 |
| 39~50 | 90~75 | 10~25 |

In some embodiments, the gradient conditions are as follows:

| Time/min | Mobile phase A/% | Mobile phase B/% |
|---|---|---|
| 0 | 80 | 20 |
| 3 | 80 | 20 |
| 6 | 75 | 25 |
| 10 | 75 | 25 |
| 15 | 70 | 30 |
| 15.5 | 63 | 37 |
| 25 | 63 | 37 |
| 29 | 62 | 38 |
| 31 | 62 | 38 |
| 31.5 | 2 | 98 |
| 39 | 2 | 98 |
| 39.5 | 80 | 20 |
| 43 | 80 | 20 |

In some embodiments, the gradient conditions are as follows:

| Time/min | Mobile phase A/% | Mobile phase B/% | Flow rate/mL·min⁻¹ |
|---|---|---|---|
| 0 | 80 | 20 | 200 |
| 3 | 80 | 20 | 200 |
| 6 | 75 | 25 | 200 |
| 10 | 75 | 25 | 200 |
| 15 | 70 | 30 | 190 |
| 15.5 | 63 | 37 | 160 |
| 25 | 63 | 37 | 160 |
| 29 | 62 | 38 | 160 |
| 31 | 62 | 38 | 160 |
| 31.5 | 2 | 98 | 200 |
| 39 | 2 | 98 | 200 |
| 39.5 | 80 | 20 | 200 |
| 43 | 80 | 20 | 200 |

In some embodiments, in step (2-1), when purifying using the preparative liquid chromatography, the chromatographic column is UniSil 10-100 C18 450mm*100mm.

In some embodiments, in step (2-1), when purifying using the preparative liquid chromatography, the detection wavelength is from about 200nm to about 365nm including, for example, 200 nm, 220 nm, 250 nm, 270 nm, 300 nm, 350 nm, or 365nm. In one embodiment, the detection wavelength is from about 200nm to 230nm.

In some embodiments, in step (2-1), when purifying using the preparative liquid chromatography, the detection wavelength is 214nm.

In some embodiments, in step (2-2), the nanofiltration is performed under conditions with a temperature of ≤30°C.

In some embodiments, the nanofiltration is achieved using a nanofiltration device.

In some embodiments, in step (2-2), the drying comprises lyophilizing.

In some embodiments, in step (2-i), the organic solvent comprises mixed solvent 1 and/or mixed solvent 2, wherein the mixed solvent 1 comprises ethyl acetate and n-hexane, and the mixed solvent 2 comprises dichloromethane and n-hexane.

In some embodiments, step (2-i) comprises sequentially extracting the preparative solution with mixed solvent 1 and mixed solvent 2, discarding the organic phases, and retaining the aqueous phase.

In some embodiments, the volume ratio of the mixed solvent 1 to the preparative solution is (1-5):1, for example, 1:1, 2:1, 3:1, 4:1, or 5:1, and in certain embodiments is 1:1.

In some embodiments, the volume ratio of the preparative solution to the mixed solvent 2 is (1-5):1, for example, 1:1, 2:1, 3:1, 4:1, or 5:1, and in certain embodiments is 5:2.

In some embodiments, in mixed solvent 1, the volume ratio of ethyl acetate to n-hexane is (1-5):1, for example, 1:1, 2:1, 3:1, 4:1, or 5:1, and in certain embodiments is 3:1.

In some embodiments, in mixed solvent 1, the volume ratio of ethyl acetate to n-hexane is (1-5):1, for example, 1:1, 2:1, 3:1, 4:1, or 5:1, and in certain embodiments is 3:1.

In some embodiments, in mixed solvent 2, the volume ratio of dichloromethane to n-hexane is (1-5):1, for example, 1:1, 2:1, 3:1, 4:1, or 5:1, and in certain embodiments is 3:1.

In some embodiments, the molar ratio of the Lewis acid to the compound represented by Formula a is at least 10:1, for example, 10:1, 15:1, 20:1, 25:1, 30:1, 35:1, 40:1, or 45:1, and in certain embodiments is 20:1.

In some embodiments, the molar ratio of the Lewis acid to the compound represented by Formula a is (10-30):1, for example, 10:1, 15:1, 20:1, 25:1, or 30:1, and in certain embodiments is 20:1.

In some embodiments, the reacting is carried out in the presence of a solvent, wherein the solvent comprises nitroalkanes (including without limitation, nitromethane, nitroethane), haloalkanes (including without limitation, dichloromethane, dichloroethane, chloroform), water, ketones (including without limitation, acetone, butanone), toluenes (including without limitation, toluene, trifluorotoluene), esters (including without limitation, ethyl acetate, dimethyl carbonate), acetonitrile. In one embodiment, the solvent comprises mixed solvents comprising two or more of the foregoing solvents in any proportion.

In certain embodiments, the solvent is selected from the group consisting of nitromethane, dimethyl carbonate, dichloromethane, mixed solvents of dichloromethane/ketone, mixed solvents of dichloromethane/water, and mixed solvents of dichloromethane/ester.

In some embodiments, the solvent is selected from the group consisting of nitromethane, dichloromethane/water, and dimethyl carbonate. As used herein, "dichloromethane/water" refers to a mixture comprising dichloromethane and water.

In some embodiments, the ratio of the mass of the compound represented by Formula a to the volume of the solvent is 1 g/(5-50 mL), for example, 1 g/5 mL, 1 g/10 mL, 1 g/15 mL, 1 g/20 mL, 1 g/25 mL, 1 g/30 mL, 1 g/35 mL, 1 g/40 mL, 1 g/45 mL, or 1 g/50 mL, and in certain embodiments is 1 g/(5-35 mL).

In some embodiments, the solvent is nitromethane.

In some embodiments, the ratio of the mass of the compound represented by Formula a to the volume of the nitromethane is 1 g/(20-50 mL), for example, 1 g/20 mL, 1 g/25 mL, 1 g/30 mL, 1 g/35 mL, 1 g/40 mL, 1 g/45 mL, or 1 g/50 mL, and in certain embodiments is 1 g/35 mL.

In some embodiments, the solvent is dichloromethane/water.

In some embodiments, the ratio of the mass of the compound represented by Formula a to the volume of the dichloromethane/water is 1 g/(10-30 mL), for example, 1 g/10 mL, 1 g/15 mL, 1 g/16 mL, 1 g/20 mL, 1 g/25 mL, or 1 g/30 mL, and in certain embodiments is 1 g/(15-16 mL).

In some embodiments, in the dichloromethane/water, the volume ratio of dichloromethane to water is (500-1000):1, for example, 500:1, 550:1, 600:1, 650:1, 700:1, 750:1, 800:1, 850:1, 900:1, 950:1, or 1000:1, and in certain embodiment is 750:1.

In some embodiments, the solvent is dimethyl carbonate.

In some embodiments, the ratio of the mass of the compound represented by Formula a to the volume of the dimethyl carbonate is 1 g/(5-30 mL), for example, 1 g/5 mL, 1 g/10 mL, 1 g/15 mL, 1 g/20 mL, 1 g/25 mL, or 1 g/30 mL, and in certain embodiment is 1 g/(5-15 mL).

In some embodiments, the reacting is carried out at a temperature of 20-110°C (e.g., 20°C, 30°C, 40°C, 50°C, 60°C, 70°C, 80°C, 90°C, 100°C, or 110°C). In some embodiments, the reacting is carried out at a temperature of 20-50°C (e.g., 20°C, 30°C, 35°C, 40°C, 45°C, or 50°C, and in certain embodiment 40°C). In some embodiments, the reacting is carried out at a temperature of 30-50°C (e.g., 30°C, 35°C, 40°C, 45°C, or 50°C, and in certain embodiment 40°C).

In some embodiments, the reacting is carried out for a duration of 20 min to 24 h (e.g., 20 min, 30 min, 40 min, 1 h, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, or 24 h). In some embodiments, the reacting is carried out for a duration of 20-120 minutes (e.g., 20 min, 25 min, 30 min, 35 min, 40 min, 45 min, 50 min, 55 min, 60 min, 70 min, 80 min, 90 min, 100 min, 110 min, or 120 min). In some embodiments, the reacting is carried out for a duration of 20-60 minutes. In some embodiments, the reacting is carried out for a duration of 30-60 minutes. In some embodiments, the reacting is carried out for 30 minutes.

In some embodiments, after reacting the compound represented by Formula a with the Lewis acid and before purifying, the method further comprises a post-treatment step.

In some embodiments, the post-treatment step comprises: concentrating the obtained reactant under reduced pressure to obtain the crude product.

In some embodiments, the post-treatment step comprises: crystallizing, filtering, and drying the obtained reactant to obtain the crude product.

In some embodiments, the concentrating under reduced pressure is carried out at a temperature of 30-55°C (e.g., 30°C, 35°C, 40°C, 45°C, 50°C, or 55°C, and in certain embodiment 45°C).

In some embodiments, the compound represented by Formula a is prepared by the following methods:
(3-1-1) reacting a compound represented by Formula b with a base (e.g., triethylamine) to obtain a compound 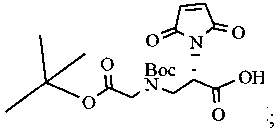 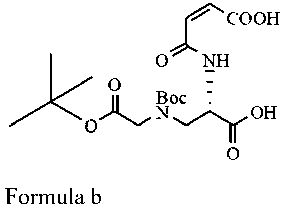
(3-1-2) reacting the compound 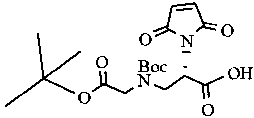 with a compound 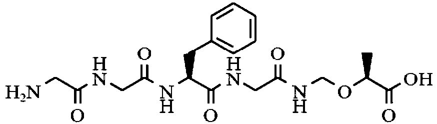 to obtain a compound 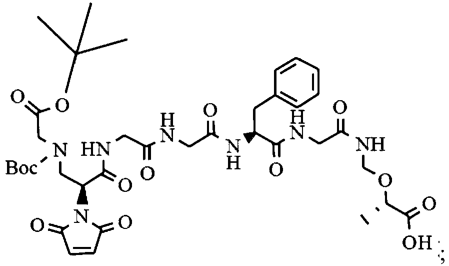
(3-1-3) reacting the compound 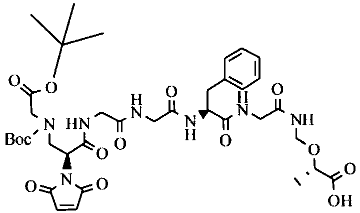 with exatecan mesylate to obtain the compound represented by Formula a;

Alternative, the compound represented by Formula a is prepared by the following methods:
(3-2-1) reacting a compound represented by Formula b with a base (e.g., triethylamine) to obtain a compound 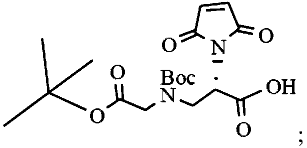 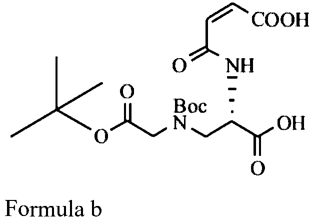
(3-2-2) reacting the compound 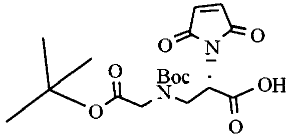 with pentafluorophenol and DCC to obtain a compound 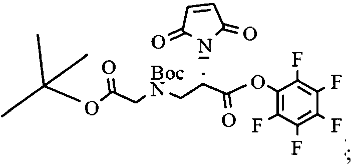
(3-2-3) reacting the compound 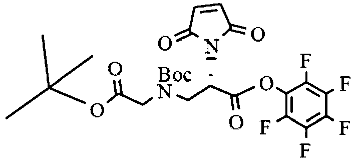 with a compound 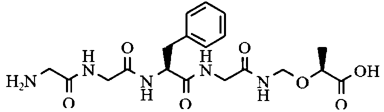 to obtain a compound 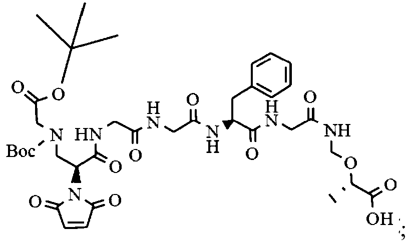
(3-2-4) reacting the compound 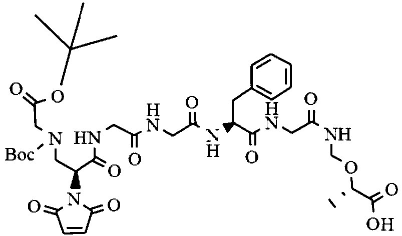 with exatecan mesylate to obtain the compound represented by Formula a.

In some embodiments, the compound represented by Formula b is prepared by the following steps:
(4-1) reacting a compound 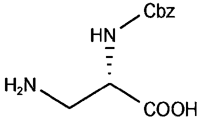 with thionyl chloride and benzyl alcohol to obtain a compound 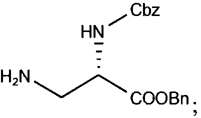
(4-2) reacting the compound 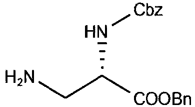 with a compound 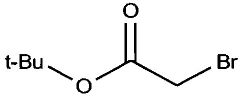 to obtain a compound 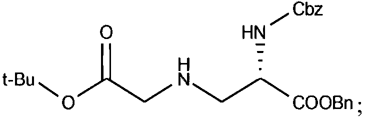
(4-3) reacting the compound 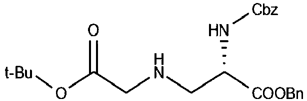 with (Boc)₂O to obtain a compound 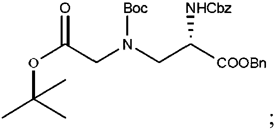
(4-4) reacting the compound 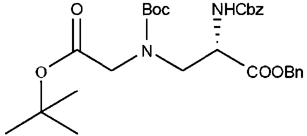 with hydrogen gas in the presence of Pd/C to obtain a compound 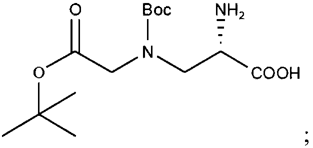
(4-5) reacting the compound 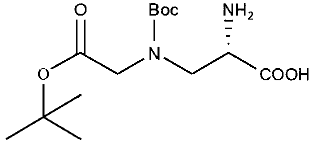 with maleic anhydride to obtain the compound represented by Formula b.

In some embodiments, the step (4-1) is carried out by the following steps:
(4-1-1) adding thionyl chloride dropwise to benzyl alcohol, followed by adding the compound 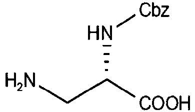 to react;
(4-1-2) adding isopropyl ether to the reactant obtained in step (4-1-1) to induce crystallization, filtering, and drying the filter cake to obtain the compound

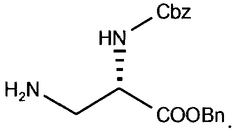

In some embodiments, step (4-4) is carried out by the following steps:
(4-4-1) reacting the compound 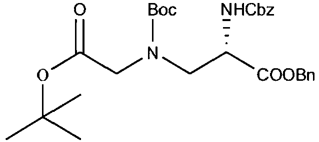 with hydrogen gas in the presence of Pd/C;
(4-4-2) filtering the reactant obtained in step (4-4-1), and concentrating the filtrate under reduced pressure to obtain a crude product;
(4-4-3) recrystallizing the crude product with isopropanol/water to obtain a crystal, which is the compound 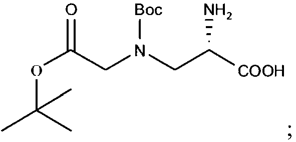
preferably, when recrystallizing, the volume ratio of isopropanol to water is (1-5):1, preferably 3:1.

In some embodiments, the step (4-5) is carried out by the following steps:
(4-5-1) reacting the compound 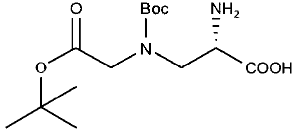 with maleic anhydride;
(4-5-2) adding n-hexane to the reactant obtained in step (4-5-1) to induce crystallization, filtering, and drying the filter cake to obtain the compound represented by Formula b.

In another aspect of the application, a method for preparing the compound represented by Formula A is also provided.

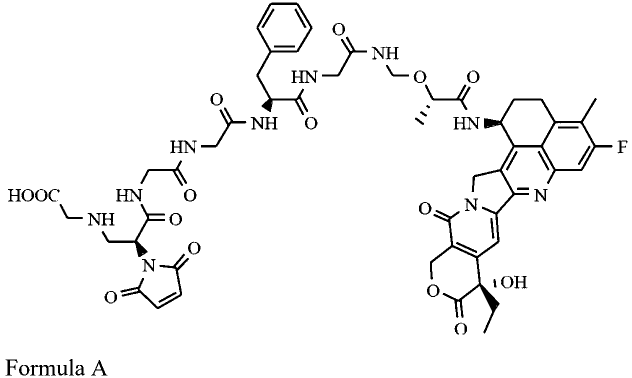

In one embodiment, the method comprises:
(1-1) reacting the compound represented by Formula a with nitromethane and zinc bromide to obtain a crude product; 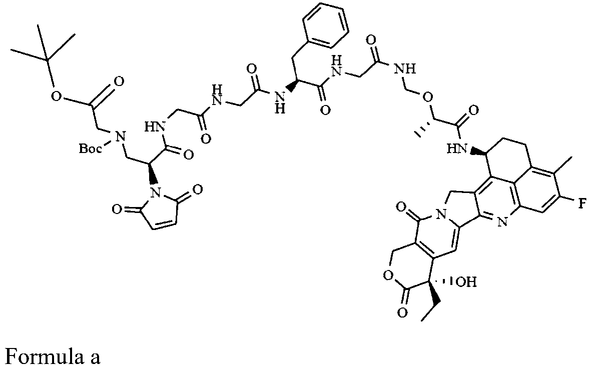
(1-2) purifying the crude product by a preparative liquid chromatography to obtain a preparative solution;
(1-3) extracting the preparative solution with an organic solvent, discarding the organic phase, and obtaining the aqueous phase;
(1-4) concentrating and drying the aqueous phase to obtain the compound represented by Formula A;
wherein, in step (1-2), when purifying by the preparative liquid chromatography, the mobile phase A is 0.2% TFA/water, and the mobile phase B is 0.1% TFA/acetonitrile; in step (1-4), the concentrating is nanofiltration concentrating.

In some embodiments, in step (1-2), gradient elution is used when purifying using the preparative liquid chromatography.

In some embodiments, the gradient conditions are as follows:

| Time/min | Mobile phase A/% | Mobile phase B/% | Flow rate/mL·min⁻¹ |
|---|---|---|---|
| 0 | 80 | 20 | 200 |
| 3 | 80 | 20 | 200 |
| 6 | 75 | 25 | 200 |
| 10 | 75 | 25 | 200 |
| 15 | 70 | 30 | 190 |
| 15.5 | 63 | 37 | 160 |
| 25 | 63 | 37 | 160 |
| 29 | 62 | 38 | 160 |
| 31 | 62 | 38 | 160 |
| 31.5 | 2 | 98 | 200 |
| 39 | 2 | 98 | 200 |
| 39.5 | 80 | 20 | 200 |
| 43 | 80 | 20 | 200 |

In some embodiments, in step (1-2), when purifying by the preparative liquid chromatography, the chromatographic column is UniSil 10-100 C18 450mm*100mm.

In some embodiments, in step (1-2), when purifying by the preparative liquid chromatography, the detection wavelength is 214nm.

In some embodiments, in step (1-4), the nanofiltration is performed under a condition with temperature ≤30°C.

In some embodiments, the nanofiltration is achieved using a nanofiltration device.

In some embodiments, in step (1-4), the drying is lyophilizing.

In some embodiments, in step (1-3), the organic solvent is mixed solvent 1 and/or mixed solvent 2, wherein the mixed solvent 1 is a mixed solvent of ethyl acetate and n-hexane, and the mixed solvent 2 is a mixed solvent of dichloromethane and n-hexane.

In some embodiments, the step (1-3) is performed by sequentially extracting the preparative solution with the mixed solvent 1 and the mixed solvent 2, discarding the organic phases, and obtaining the aqueous phase.

In some embodiments, the volume ratio of the mixed solvent 1 to the preparative solution is (1-5):1, for example, 1:1, 2:1, 3:1, 4:1, or 5:1, and in certain embodiments is 1:1.

In some embodiments, the volume ratio of the preparative solution to the mixed solvent 2 is (1-5):1, for example, 1:1, 2:1, 3:1, 4:1, or 5:1, and in certain embodiment is 5:2.

In some embodiments, in the mixed solvent 1, the volume ratio of ethyl acetate to n-hexane is (1-5):1, for example, 1:1, 2:1, 3:1, 4:1, or 5:1, and in certain embodiment is 3:1.

In some embodiments, in the mixed solvent 2, the volume ratio of dichloromethane to n-hexane is (1-5):1, for example, 1:1, 2:1, 3:1, 4:1, or 5:1, and in certain embodiment is 3:1.

In some embodiments, in step (1-1), the molar ratio of the zinc bromide to the compound represented by Formula a is (10-30):1, for example, 10:1, 15:1, 20:1, 25:1, or 30:1, and in certain embodiment is 20:1.

In some embodiments, in step (1-1), the ratio of the mass of the compound represented by Formula a to the volume of the nitromethane is 1 g/(20-50 mL), for example, 1 g/20 mL, 1 g/25 mL, 1 g/30 mL, 1 g/35 mL, 1 g/40 mL, 1 g/45 mL, or 1 g/50 mL, and in certain embodiment is 1 g/35 mL.

In some embodiments, in step (1-1), the reacting is carried out at a temperature of 30-50°C (e.g., 30°C, 35°C, 40°C, 45°C, or 50°C, and in certain embodiment 40°C).

In some embodiments, in step (1-1), the reacting is carried out for a duration of 20-60 min (e.g., 20 min, 25 min, 30 min, 35 min, 40 min, 45 min, 50 min, 55 min, or 60 min, and in certain embodiment 30 min).

In some embodiments, in step (1-1), the reacting of the compound represented by Formula a with nitromethane and zinc bromide is carried out as follows:
(1-1-1) mixing the compound represented by Formula a with nitromethane, dissolving, and then adding zinc bromide to react;
(1-1-2) concentrating the reactant obtained in step (1-1-1) under reduced pressure to obtain the crude product.

In some embodiments, the concentrating under reduced pressure is carried out at a temperature of 30-55°C (e.g., 30°C, 35°C, 40°C, 45°C, 50°C, or 55°C, and in certain embodiment 45°C).

In some embodiments, the compound represented by Formula a is prepared by the following methods:
(2-1) reacting a compound represented by Formula b with triethylamine to obtain a compound 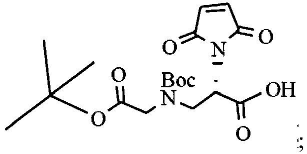 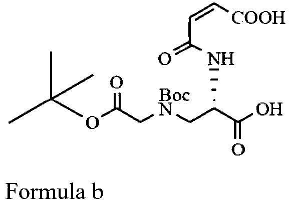
(2-2) reacting the compound 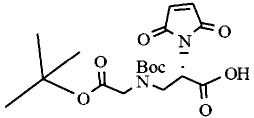 with pentafluorophenol and DCC to obtain a compound 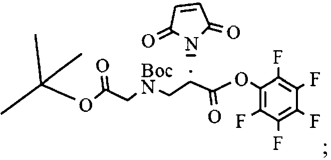
(2-3) reacting the compound 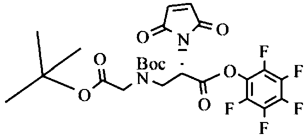 with a compound 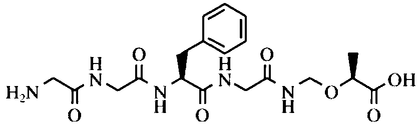 to obtain a compound 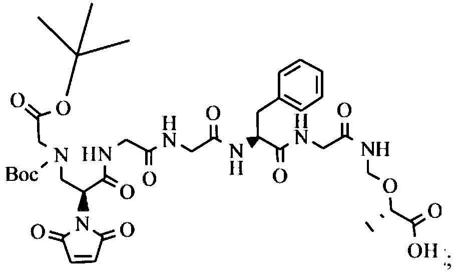
(2-4) reacting the compound 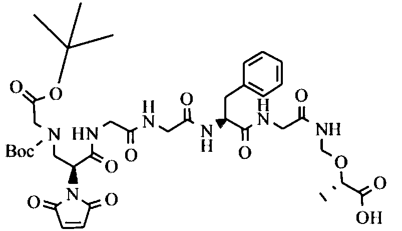 with exatecan mesylate to obtain the compound represented by Formula a.

In some embodiments, the compound represented by Formula b is prepared by the following methods:
(3-1) reacting a compound 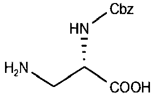 with thionyl chloride and benzyl alcohol to obtain a compound 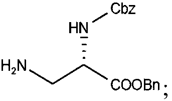
(3-2) reacting the compound 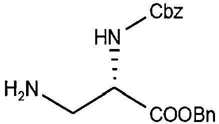 with a compound 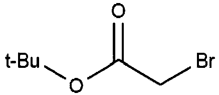 to obtain a compound 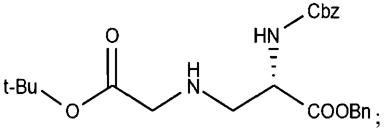
(3-3) reacting the compound 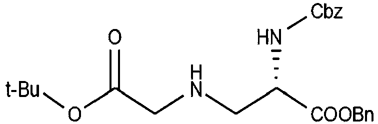 with (Boc)₂O to obtain a compound 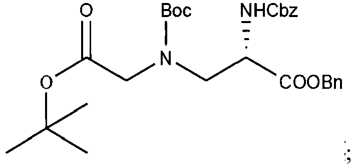
(3-4) reacting the compound 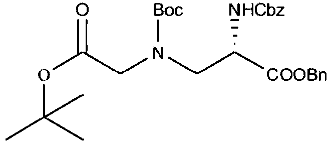 with hydrogen gas in the presence of Pd/C to obtain a compound 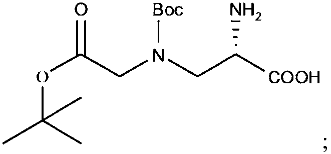
(3-5) reacting the compound 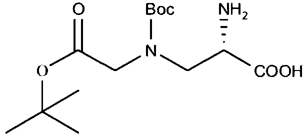 with maleic anhydride to obtain the compound represented by Formula b.

In some embodiments, the step (3-1) is performed as follows:
(3-1-1) adding thionyl chloride dropwise to benzyl alcohol, followed by adding the compound 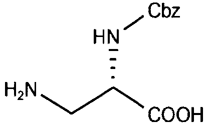 to react;
(3-1-2) adding isopropyl ether to the reactant obtained in step (3-1-1) to induce crystallization, filtering, and drying the filter cake to obtain the compound

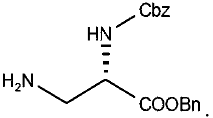

In some embodiments, the step (3-4) is performed as follows:
(3-4-1) reacting the compound 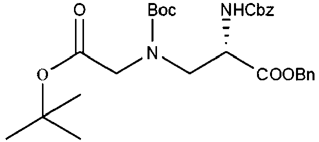 with hydrogen gas in the presence of Pd/C;
(3-4-2) filtering the reactant obtained in step (3-4-1), and concentrating the filtrate under reduced pressure to obtain a crude product;
(3-4-3) recrystallizing the crude product with isopropanol/water to obtain a crystal, which is the compound

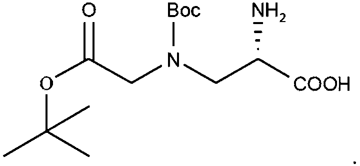

In some embodiments, when recrystallizing, the volume ratio of isopropanol to water is (1-5):1, for example, 1:1, 2:1, 3:1, 4:1, or 5:1, and in certain embodiment is 3:1.

In some embodiments, the step (3-5) is performed as follows:
(3-5-1) reacting the compound 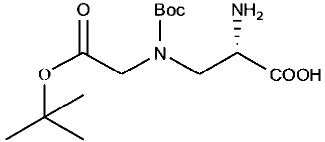 with maleic anhydride;
(3-5-2) adding n-hexane to the reactant obtained in step (3-5-1) to induce crystallization, filtering, and drying the filter cake to obtain the compound represented by Formula b.

### BRIEF DESCRIPTION OF THE DRAWINGS

FIG. 1 shows the impurity detection results of Step 1 of Example 1;
FIG. 2 shows the impurity detection results of Step 1 of Example 2;
FIG. 3 shows the impurity detection results of Step 1 of Example 3;
FIG. 4 shows the impurity detection results of Step 1 of Comparative Example 1;
FIG. 5 depicts the impurity detection results of Step 2 of Example 1;
FIG. 6 depicts the impurity detection results of Step 2 of Comparative Example 2;
FIG. 7 depicts the impurity detection results of Step 2 of Comparative Example 3;
FIG. 8 depicts the impurity detection results of Step 2 of Comparative Example 4;
FIG. 9 depicts the impurity detection results of Step 2 of Comparative Example 5;
FIG. 10 depicts the impurity detection results of Step 2 of Comparative Example 6;
FIG. 11 depicts the impurity detection results of Step 2 of Comparative Example 7;
FIG. 12 depicts the impurity detection results of Step 2 of Comparative Example 8; and
FIG. 13 depicts the impurity detection results of Step 2 of Comparative Example 9.

### DETAILED DESCRIPTION

The embodiments of the present application are described below with reference to the accompanying drawings and examples. The drawings and examples are provided for purposes of illustration and are not intended to limit the scope of the claims. Those skilled in the art will recognize that various modifications, variations, and combinations may be made without departing from the scope of the present application.

Further aspects and features of the present application will become apparent from the following detailed description.

In the following experimental steps, unless otherwise specified, all raw materials are commercially available.

### Preliminary Example 1:

### Preparation of Compound

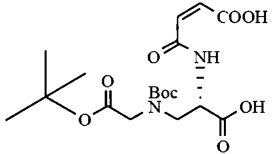

### Synthetic Scheme:

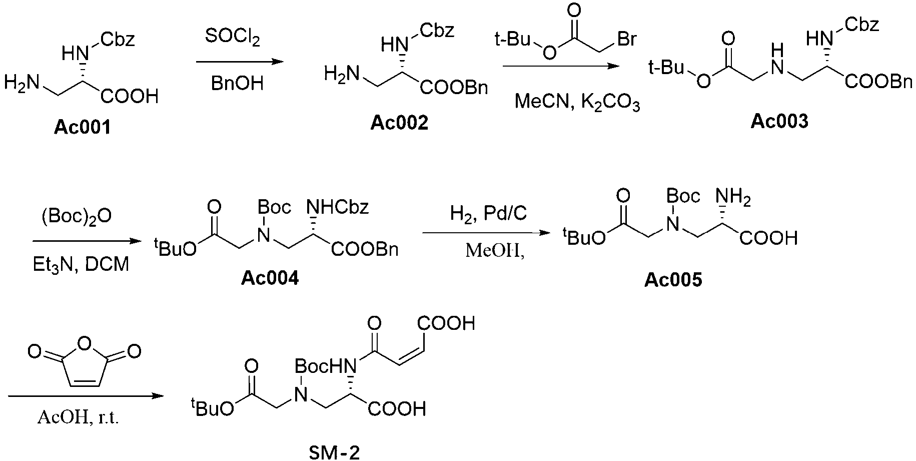

### Step 1: Ac002

2.8L of benzyl alcohol was added to a 5L three-necked flask, cooled to below 10°C, and added with thionyl chloride (36mL, 0.50mol, 2.0eq) dropwise. After the addition was complete, the mixture was warmed to room temperature and reacted for 2 hours. Then, 3-amino-N-Cbz-L-alanine (60g, 0.25mol, 1.0eq) was added, and reacted at room temperature for 16 hours. When the raw material **(Ac001)** had essentially disappeared as monitored by TLC, the reaction was terminated. 12L of isopropyl ether was added to the system, crystallization was carried out for 2 hours, followed by filtration, and then the filter cake was dried at 45°C with a forced airflow to obtain 55.2g of **Ac002** with a yield of 60.2%.

### Step 2: Ac003

**Ac002** (55g, 0.15mol, 1.0eq), 300mL of acetonitrile, and 33mL of purified water were added to a 1L reaction flask. After stirring and dissolving, potassium carbonate (31.2g, 0.225mol, 1.5eq) was added, followed by dropwise addition of tert-butyl bromoacetate (26.42g, 0.135mol, 0.9eq). After the dropwise addition was complete, the mixture was reacted at room temperature for 20 hours. When the raw material **(Ac002)** had essentially disappeared as monitored by TLC, the reaction was terminated. Purified water (1.5L) was added to the system, and extraction was carried out using ethyl acetate (1L). The layers were separated, and the organic layer was washed with 10% sodium chloride solution, dried over anhydrous sodium sulfate, filtered, and concentrated under reduced pressure to obtain a crude product. The crude product was purified by column chromatography (petroleum ether/ethyl acetate) to obtain 44.4g of **Ac003** with a yield of 66%.

### Step 3: Ac004

**Ac003** (44.2 g, 0.1 mol, 1.0 eq) and 100 mL of dichloromethane were added to a 500 mL reaction flask and stirred to dissolve. Triethylamine (15 mL, 0.11 mol, 1.1 eq) was then added, followed by dropwise addition of Boc anhydride (22.9 g, 0.105 mol, 1.05 eq). The mixture was reacted at room temperature for 20 hours. When the raw material **(Ac003)** had essentially disappeared as monitored by TLC, the reaction was terminated. Purified water (100 mL) was added to the system, and the layers were separated. The aqueous layer was extracted with dichloromethane (40 mL). The organic layers were combined, washed with purified water, dried over anhydrous sodium sulfate, filtered, and concentrated under reduced pressure to obtain 55.3 g of yellow oily substance, which was directly used in the next step.

### Step 4: Ac005

The yellow oily substance (55.3g) from Step 3 and 200mL of methanol were added to a 1L single-necked flask and stirred to dissolve. 5% Pd/C (8.3g, 15% m/m) was then added, and hydrogenation was carried out for 4 hours. When the raw material (**Ac004**) had essentially disappeared as monitored by TLC, the reaction was terminated. The reaction solution was filtered, and the filtrate was concentrated under reduced pressure to obtain a crude product. The crude product was recrystallized by using isopropanol/water (3/1 by volume) to give 18.4 g of **Ac005** with a yield of 58% (two steps).

### Step 5: SM-2

**Ac005** (18 g, 0.056 mol, 1.0 eq), 100 mL of dichloromethane, and 17 mL of glacial acetic acid were added to a 500 mL single-necked flask and stirred to dissolve. Maleic anhydride (6 g, 0.062 mol, 1.1 eq) was then added and reacted at room temperature for 4 hours. When the raw material (**Ac005**) had essentially disappeared as monitored by TLC, the reaction was terminated. n-Hexane (80 mL) was added to the system, the mixture was cooled to 10 °C, and crystallized for 2 hours. After filtering, the filter cake was dried at 50 °C with forced airflow for 4 hours to give 20.6 g of **SM-2** with a yield of 87%.

### Preliminary Example 2:

### Preparation of Compound

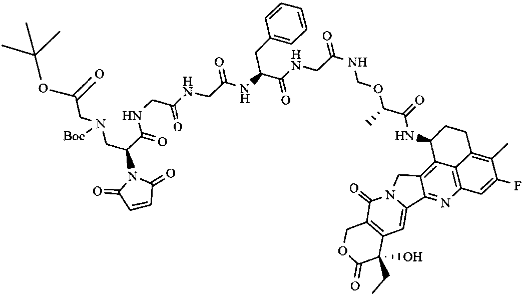

### 1. Synthesis of Compound M3:

### Synthetic Scheme:

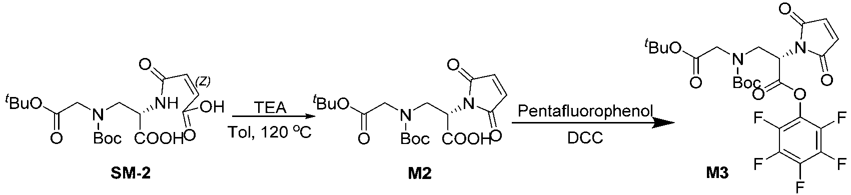

### Step 1: Compound M2

**SM-2** (40 g, 96 mmol, 1.0 eq), triethylamine (26.7 mL, 2.0 eq), and toluene (400 mL) were added to a 1000 mL single-necked flask. The mixture was refluxed and reacted at 120 °C for 2 hours. The reaction was monitored by TLC until almost complete. The system was cooled to 50 °C and the solvent was removed under reduced pressure. The residual mixture was dissolved in ethyl acetate (150 mL) and water (40 mL), adjusted to pH 2-3 with 1M HCl under stirring in an ice bath. The layers were separated. The aqueous layer was extracted again with ethyl acetate. The organic layers were combined, dried over anhydrous sodium sulfate, filtered, and then concentrated to obtain a pale yellow oily crude product. The crude product was purified by column chromatography (DCM:MeOH = 40:1) to obtain compound **M2** (26.6 g); LC-MS: [M+H]⁺ = 399.3.

### Step 2: Compound M3

In a 1000 mL single-necked flask, compound **M2** (26.5 g, 60.5 mmol, 1.0 eq), pentafluorophenol (12.2 g, 66.5 mmol, 1.1 eq), DCC (13.7 g, 66.5 mmol, 1.1 eq), and THF (300 mL) were added. The mixture was reacted at room temperature for 30 minutes (monitored by TLC). Insoluble matter was filtered off. The reaction solution was then directly purified by preparative liquid chromatography. The resulting preparative solution was concentrated in a water bath at 35 °C under reduced pressure using a water pump to remove acetonitrile, and then lyophilized to obtain compound **M3** (31.5 g) with a yield of 64%; LC-MS: [M+H]⁺ = 565.1.

### 2. Synthesis of Compound 5e:

### Synthetic Schemes:

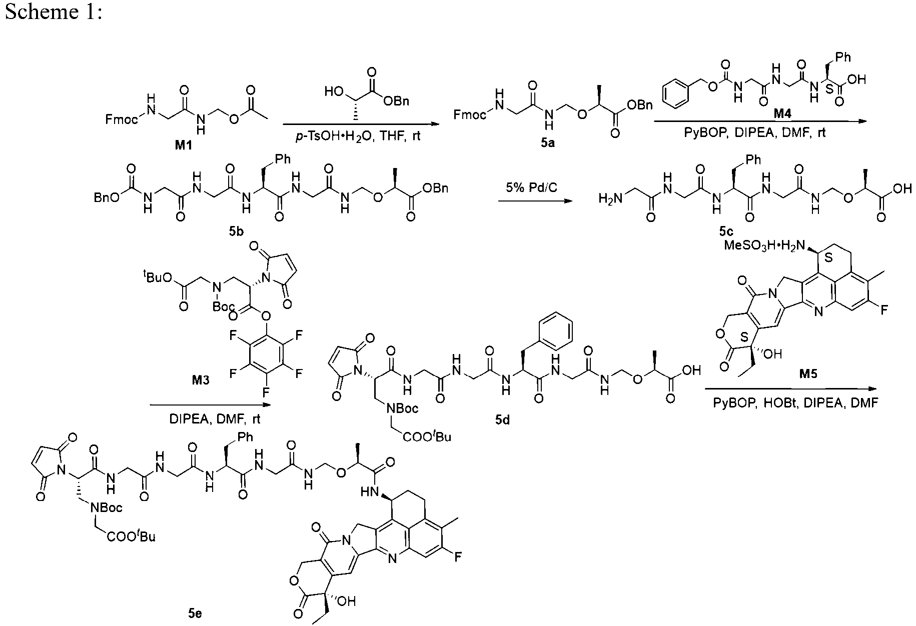

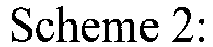

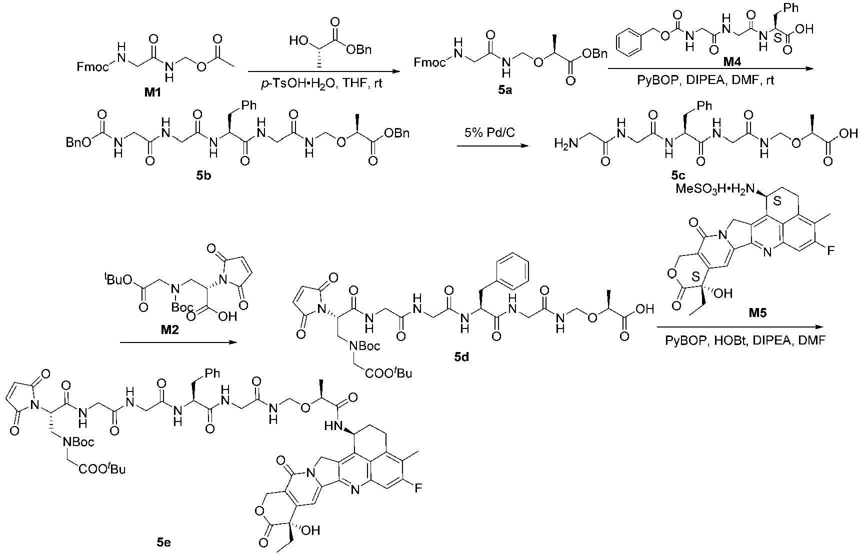

### Step 1: Compound 5a

To a 25 mL single-necked flask, **M1** (500 mg, 1.4 mmol, 1.0 eq), p-toluenesulfonic acid monohydrate (26 mg, 0.1 mmol, 0.1 eq), and 10 mL of THF were added. After stirring, the mixture was cooled to 0 °C, and then *L*-benzyl lactate (1.2 g, 7.0 mmol, 5 eq) was slowly added. After the addition was complete, the mixture was warmed to room temperature and reacted. The reaction was monitored by TLC. After the reaction was complete, saturated NaHCO₃ solution was added, and the mixture was extracted with ethyl acetate, dried over anhydrous sodium sulfate, filtered, and concentrated. The residue was purified by reverse-phase column chromatography to obtain **5a** (400 mg).

LC-MS: [M+NH₄]⁺ = 506.2.

¹H NMR (400 MHz, CDCl₃/CD₃OD): 1.39 (3H, d, *J* = 6.8 Hz), 3.78 (2H, t, *J* = 4.0 Hz), 4.17-4.27 (2H, m), 4.42 (2H, d, *J* = 4.0 Hz), 4.72-4.85 (2H, m), 5.11-5.58 (2H, m), 5.43 (1H, s), 7.06 (1H, t, *J* = 8.0 Hz), 7.25-7.33 (6H, m), 7.38 (2H, t, J=8.0 Hz), 7.57 (2H, d, *J* = 8.0 Hz), 7.75 (2H, d, *J* = 8.0 Hz).

### Step 2: Compound 5b

To a 25 mL single-necked flask, compound **5a** (400 mg, 0.8 mmol, 1.0 eq) and 4 mL of DMF were added. After stirring thoroughly, the mixture was cooled to 0 °C, and then DBU (137 mg, 0.9 mmol, 1.1 eq) was added slowly. After the addition was complete, the mixture was warmed to room temperature and reacted. The reaction was monitored by TLC. After the reaction was complete, the product was recorded as reaction solution ①.

To another 25 mL single-necked flask, **M4** (372 mg, 0.9 mmol, 1.1 eq), PyBOP (852 mg, 1.6 mmol, 2.0 eq), and 3 mL of DMF were added, and stirred at room temperature for 5 minutes. Then, the reaction solution ① was added and reacted at room temperature. The reaction was monitored by HPLC. After the reaction was complete, the reaction solution was purified by HPLC to obtain compound **5b** (326 mg); LC-MS: [M+NH₄]⁺=679.2.

### Step 3: Compound 5c

**5b** (4.0 g, 6.05 mmol, 1.0 eq) was added to a 100 mL single-necked flask and dissolved in DMF (60 mL). Then, 5% Pd/C (4 g) was added, and hydrogenation was carried out at room temperature for 4 hours (the reaction progress was monitored by HPLC). The Pd/C was then filtered off, and the filtrate, without concentration, was placed directly in an ice-water bath (approximately 0 °C) for later use.

### Step 4: Compound 5d

Scheme 1: The crude product **5c** was placed in an ice-water bath, added with DIPEA (1.1 mL, 1.1 eq), and then added with compound **M3** (3.4 g, 6.05 mmol). After the addition was complete, the mixture was warmed to room temperature and reacted for 2 hours. After the reaction was completed according to the monitoring by HPLC, the reaction solution was purified by HPLC to obtain a preparative solution, which was then lyophilized to obtain **5d** (3.15 g); LC-MS: [M-H]⁻ = 816.3.

Scheme 2: Compound **M2** (2.4 g, 6.05 mmol, 1.0 eq) was added to a single-necked flask and dissolved in acetonitrile (24 mL). The mixture was cooled to -10 ± 5 °C, and added with EEDQ (1.49 g, 6.05 mmol, 1.0 eq). The mixture was maintained at this temperature and reacted for 2 hours. Then, the crude product **5c** was added to the system, and the mixture was maintained at this temperature and reacted for 1 hour. The reaction was monitored by HPLC until completion. The reaction solution was purified by high-performance liquid chromatography to obtain a preparative solution. The preparative solution was extracted with dichloromethane and concentrated to obtain **5d** (3.0 g).

### Step 5: Compound 5e

To a 100 mL single-necked flask, **5d** (2.07 g, 2.53 mmol, 1.0 eq), exatecan mesylate **M5** (1.35 g, 2.53 mmol, 1.0 eq), PyBOP (1.98 g, 3.79 mmol, 1.5 eq), HOBt (0.51 g, 3.79 mmol, 1.5 eq), and DMF (40 mL) were added. Then, DIPEA (1.05 mL, 1.5 eq) was added under ice-water bath conditions, and the mixture was warmed to room temperature and reacted for 2 hours (monitored by HPLC). The reaction solution was directly purified by preparative liquid chromatography. The resulting preparative solution was concentrated in a water bath at 35 °C under reduced pressure using a water pump to remove acetonitrile, and then lyophilized to obtain compound **5e** (1.92 g) with a yield of 61%; LC-MS: [M+H]⁺ = 1235.4.

### Example 1:

### Preparation of the Target Compound

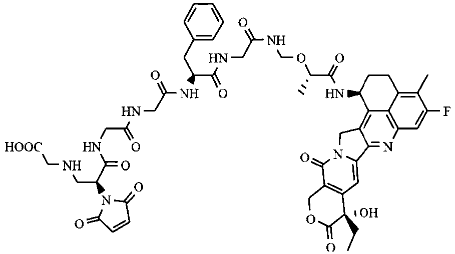

### Synthetic Scheme:

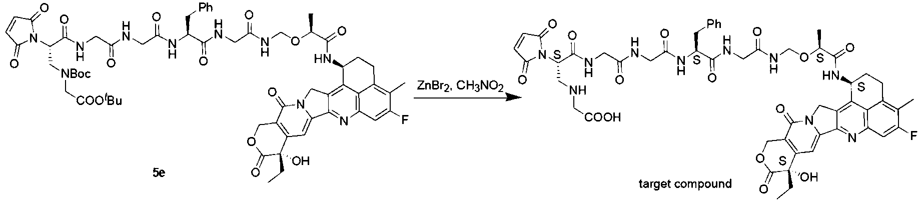

Step 1: Compound **5e** (1.0 g, 0.8 mmol, 1.0 eq) and 35 mL of nitromethane were added to a 100 mL single-necked flask and dissolved. Then zinc bromide (3.64 g, 16 mmol, 20.0 eq) was added. The mixture was reacted in an oil bath at 40 °C (preheated for stability) for 30 minutes (monitored by HPLC). The mixture was then concentrated in a water bath at 45 °C under reduced pressure using a water pump to remove the nitromethane, yielding a yellow solid residue.

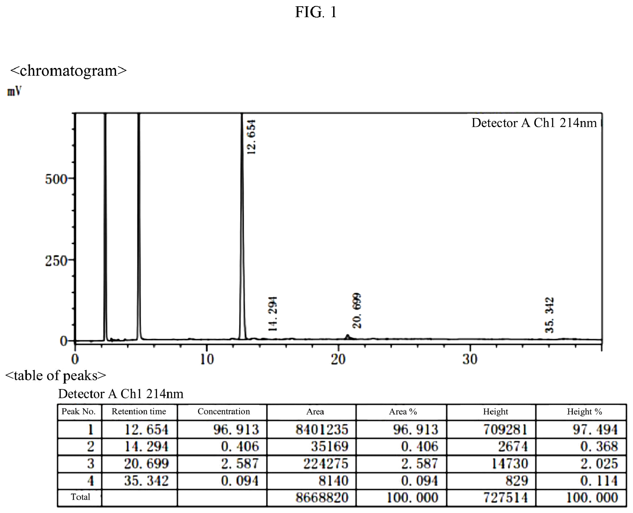

Method for detection of impurities:
The target compound was analyzed for impurities using HPLC. The results are shown in Table 1 and Figure 1.
Instrument: High performance liquid chromatography (HPLC)
Column: YMC-Triart C18-5µm-12nm 4.6mm*250mm
Mobile phase A: 0.05% TFA/water (0.5mL of TFA was added to 1L of purified water)
Mobile phase B: 0.05% TFA/acetonitrile (0.5mL of TFA was added to 1L of acetonitrile)
Detection wavelength: 214nm
Flow rate: 1mL/min
Column temperature: 30°C
Gradient conditions:

| Time/min | Mobile phase A/% | Mobile phase B/% |
|---|---|---|
| 0 | 70 | 30 |
| 20 | 55 | 45 |
| 20.01 | 2 | 98 |
| 30 | 2 | 98 |
| 30.01 | 70 | 30 |
| 40 | 70 | 30 |

Step 2: After purification by preparative liquid chromatography under acidic conditions (mobile phase: 0.2% TFA/water - 0.1% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated at low temperature (≤30°C) using a nanofiltration device and lyophilized to obtain the target compound.

LC-MS: [M+H]⁺=1079.4;
¹H NMR (400 MHz, DMSO-d6) δ 9.39 - 9.02 (m, 1H), 8.70 (t, *J* = 6.5 Hz, 1H), 8.64 (t, *J* = 5.7 Hz, 1H), 8.56 (d, *J* = 8.8 Hz, 1H), 8.34 (t, *J* = 5.7 Hz, 1H), 8.16 (d, *J* = 8.2 Hz, 1H), 8.01 (t, *J* = 5.5 Hz, 1H), 7.71 (d, *J* = 10.9 Hz, 1H), 7.3 0 (s, 1H), 7.28 - 7.15 (m, 4H), 7.14 (s, 2H), 5.53 (dd, *J* = 14.5 , 6.4 Hz, 1H), 5.49 - 5.34 (m, 2H), 5.22 (d, *J* = 18.8 Hz, 1H), 5 .09 (d, *J* = 18.7 Hz, 1H), 5.03 (dd, *J* = 9.6, 3.9 Hz, 1H), 4.73 ( dd, *J* = 9.9, 6.9 Hz, 1H), 4.59 (dd, *J* = 10.1, 6.5 Hz, 1H), 4.49 ( ddd, *J* = 13.2, 8.6, 4.4 Hz, 1H), 4.14 (dd, *J* = 13.3, 6.6 Hz, 2H) , 3.93 (s, 2H), 3.84 (dd, *J* = 16.5, 6.3 Hz, 1H), 3.76 (dd, *J* = 1 6.9, 5.7 Hz, 2H), 3.70 (d, *J* = 5.2 Hz, 2H), 3.60 (dd, *J* = 16.7, 5.4 Hz, 1H), 3.52 (dd, *J* = 16.4, 5.1 Hz, 1H), 3.45 (dd, *J* = 12.8 , 10.1 Hz, 1H), 3.25 - 3.15 (m, 1H), 3.14 - 3.05 (m, 1H), 3.01 ( dd, *J* = 13.7, 4.1 Hz, 1H), 2.73 (dd, *J* = 13.5, 9.8 Hz, 1H), 2.54 - 2.47 (m, 1H), 2.33 (s, 2H), 2.17 (d, *J* = 5.5 Hz, 2H), 1.91 - 1.79 (m, 2H), 1.33 (d, *J* = 6.6 Hz, 2H), 0.87 (t, *J* = 7.3 Hz, 2H).

The specific conditions for preparative liquid chromatography were as follows:
Instrument: Preparative liquid chromatography
Column: UniSil 10-100 C18 450mm*100mm
Mobile phase A: 0.2% TFA/water (2mL of TFA was added to 1L of purified water)
Mobile phase B: 0.1% TFA/acetonitrile (1mL of TFA was added to 1L of acetonitrile)
Detection wavelength: 214nm
Gradient conditions:

| Time/min | Mobile phase A/% | Mobile phase B/% | Flow rate/mL·min⁻¹ |
|---|---|---|---|
| 0 | 80 | 20 | 200 |
| 3 | 80 | 20 | 200 |
| 6 | 75 | 25 | 200 |
| 10 | 75 | 25 | 200 |
| 15 | 70 | 30 | 190 |
| 15.5 | 63 | 37 | 160 |
| 25 | 63 | 37 | 160 |
| 29 | 62 | 38 | 160 |
| 31 | 62 | 38 | 160 |
| 31.5 | 2 | 98 | 200 |
| 39 | 2 | 98 | 200 |
| 39.5 | 80 | 20 | 200 |
| 43 | 80 | 20 | 200 |

### Method for detection of impurities:

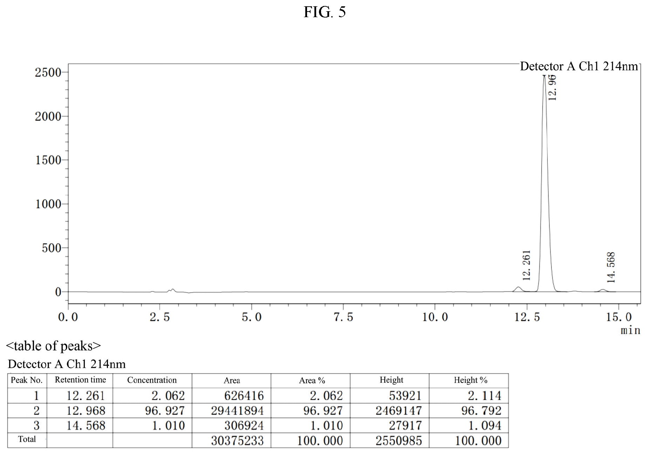

The target compound was subjected to impurity detection by HPLC. The results are shown in Table 2 and Figure 5 below.

The HPLC detection conditions for impurity detection were as follows:
Instrument: High performance liquid chromatography
Column: YMC-Triart C18-5µm-12nm 4.6mm*250mm
Mobile phase A: 0.05% TFA/water (0.5mL of TFA was added to 1L of purified water)
Mobile phase B: 0.05% TFA/acetonitrile (0.5mL of TFA was added to 1L of acetonitrile)
Detection wavelength: 214nm
Flow rate: 1mL/min
Column temperature: 30°C
Gradient conditions:

| Time/min | Mobile phase A/% | Mobile phase B/% |
|---|---|---|
| 0 | 70 | 30 |
| 20 | 55 | 45 |
| 20.01 | 2 | 98 |
| 30 | 2 | 98 |
| 30.01 | 70 | 30 |
| 40 | 70 | 30 |

### Example 2:

### Synthetic Scheme:

Compared to Example 1, the difference was only in step 1, and step 2 was the same as in Example 1. Specifically, in step 1: Compound **5e** (1.0 g, 0.8 mmol, 1.0 eq) and 15 mL of dichloromethane were added to a 100 mL single-necked flask, then 20 µL of water was added for dissolution, followed by the addition of zinc bromide (3.64 g, 16 mmol, 20.0 eq). The mixture was heated and reacted in an oil bath at 40 °C (preheated for stability) for 1 hour (monitored by HPLC), and then concentrated in a water bath at 45 °C under reduced pressure using a water pump to remove the dichloromethane, yielding a yellow solid residue.

### Impurity detection method of step 1:

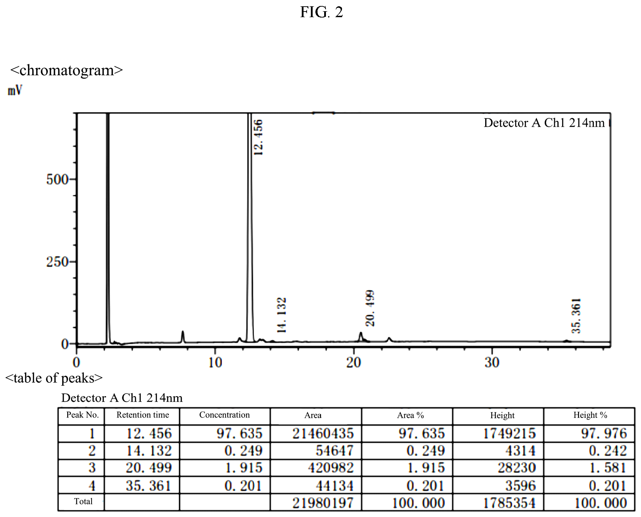

The method for detection of impurities in step 1 was the same as in Example 1. The results are shown in Table 1 and Figure 2.

### Example 3:

### Synthetic Scheme:

Compared to Example 1, the difference was only in step 1, and step 2 was the same as in Example 1. Specifically, in step 1: Compound **5e** (1.0 g, 0.8 mmol, 1.0 eq) and 15 mL of dimethyl carbonate were added to a 100 mL single-necked flask. After dissolution, zinc bromide (3.64 g, 16 mmol, 20.0 eq) was added. The mixture was heated and reacted in an oil bath at 40 °C (preheated for stability) for 30 minutes (monitored by HPLC). After the reaction was complete, 20 mL of dichloromethane was added for crystallization. After crystallization, the mixture was filtered and dried to obtain a crude product solid.

### Impurity detection method of step 1:

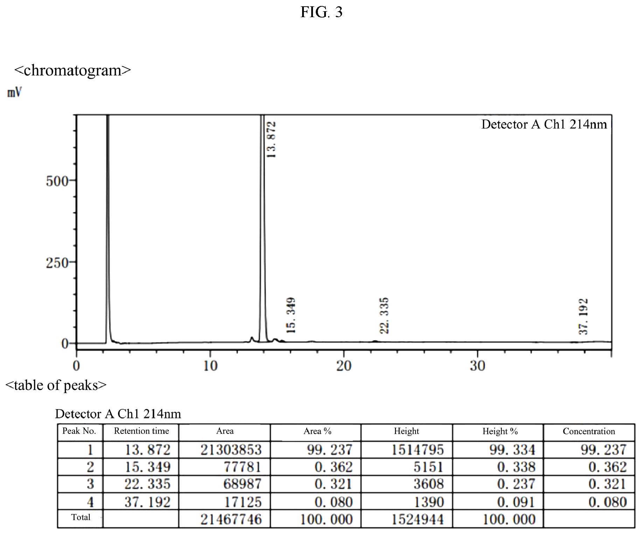

The method for detection of impurities in step 1 was the same as in Example 1. The results are shown in Table 1 and Figure 3.

### Comparative Example 1:

### Synthetic route:

Compared with Example 1, the difference was only in step 1, and the second step was the same as in Example 1. Specifically, in step 1: Compound **5e** (1.0 g, 0.8 mmol, 1.0 eq) and 10 mL of dichloromethane were added to a 100 mL single-necked flask. After dissolution, 1 mL of trifluoroacetic acid was added, and reacted at room temperature for 30 minutes (monitored by HPLC). After the reaction was complete, the mixture was concentrated under reduced pressure to obtain a crude product.

### Impurity detection method of step 1:

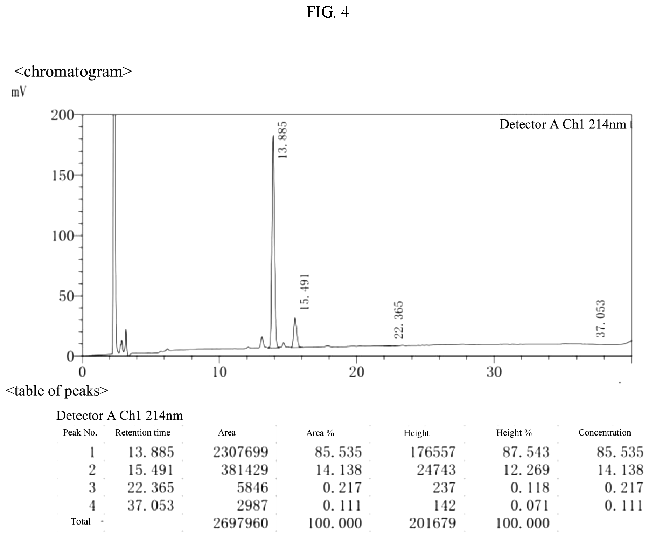

The method for detection of impurities in step 1 was the same as in Example 1. The results are shown in Table 1 and Figure 4 below.

**Table 1: Detection results of impurities**

| No. | Impurity status (HPLC peak area ratio) | | | |
|---|---|---|---|---|
| | Target compound | Monodeprotection product | Formula a | Drug unit |
| Example 1 | 96.91% | 2.59% | 0.09% | 0.41% |
| Example 2 | 97.64% | 1.92% | 0.20% | 0.25% |
| Example 3 | 99.24% | 0.32% | 0.08% | 0.36% |
| Comparative Example 1 | 85.54% | 0.22% | 0.11% | 14.14% |

From the comparison of the results of Examples 1-3 and Comparative Example 1, it could be clearly seen that in the production process of the target compound, only the preparation method sought to be protected by the application could meet the product quality standards (monodeprotection product must be controlled below 10%, compound a must be controlled below 2%, and drug unit must be controlled below 5%), and even far exceed the quality standards (drug unit was as low as 2% or below).

### Comparative Example 2:

### Synthetic Scheme:

Compared to Example 1, step 1 was the same as in Example 1, and the difference was only in step 2. Specifically, in step 2: after purification by preparative liquid chromatography (mobile phase: pure water-acetonitrile), a preparative solution of the target compound was obtained. For the specific conditions of the purification by preparative liquid chromatography, except that the mobile phase A was replaced with pure water and the mobile phase B was replaced with acetonitrile, the other specific conditions for the purification by preparative liquid chromatography were the same as in Example 1.

### Impurity detection method of step 2:

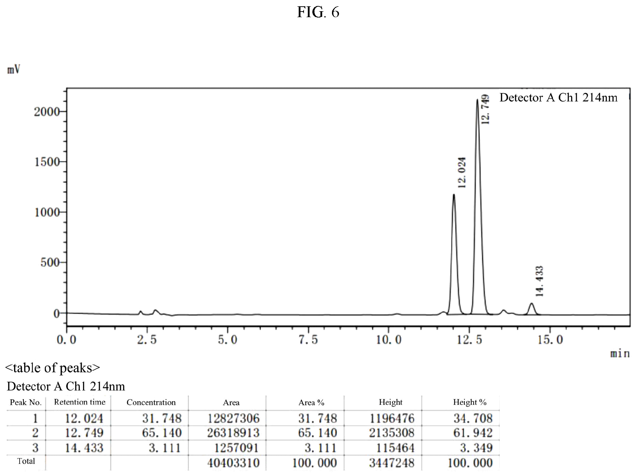

The method for detection of impurities in step 2 was the same as in Example 1. The results are shown in Table 2 and Figure 6 below.

### Comparative Example 3:

### Synthetic Scheme:

Compared to Example 1, step 1 was the same as in Example 1, and the difference was only in step 2. Specifically, in step 2: after purification by preparative liquid chromatography under acidic conditions (mobile phase: 0.1% TFA/water-acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated at low temperature (≤30°C) using a nanofiltration device and lyophilized to obtain the target compound. For the specific conditions of the purification by preparative liquid chromatography, except that the mobile phase A was replaced with 0.1% TFA/water and the mobile phase B was replaced with acetonitrile, the other specific conditions for the purification by preparative liquid chromatography were the same as in Example 1.

### Impurity detection method of step 2:

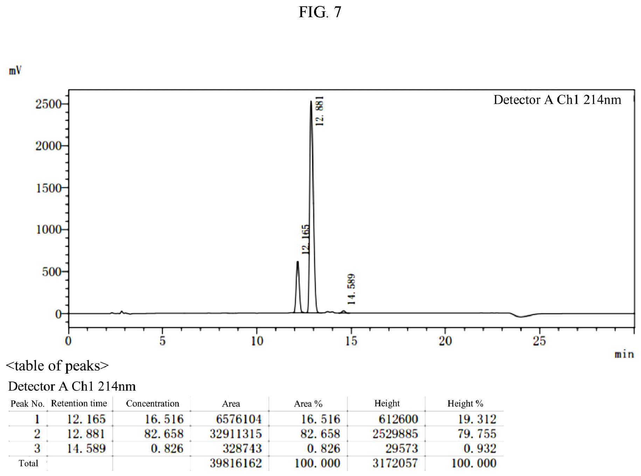

The method for detection of impurities in step 2 was the same as in Example 1. The results are shown in Table 2 and Figure 7.

### Comparative Example 4:

### Synthetic Scheme:

Compared to Example 1, step 1 was the same as in Example 1, and the difference was only in step 2. Specifically, in step 2: after purification by preparative liquid chromatography under acidic conditions (mobile phase: 0.1% TFA/water-acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated in a water bath at 35 °C under reduced pressure using an oil pump, and lyophilized to obtain the target compound. For the specific conditions of the purification by preparative liquid chromatography, except that the mobile phase A was replaced with 0.1% TFA/water and the mobile phase B was replaced with acetonitrile, the other specific conditions for the purification by preparative liquid chromatography were the same as in Example 1.

### Impurity detection method of step 2:

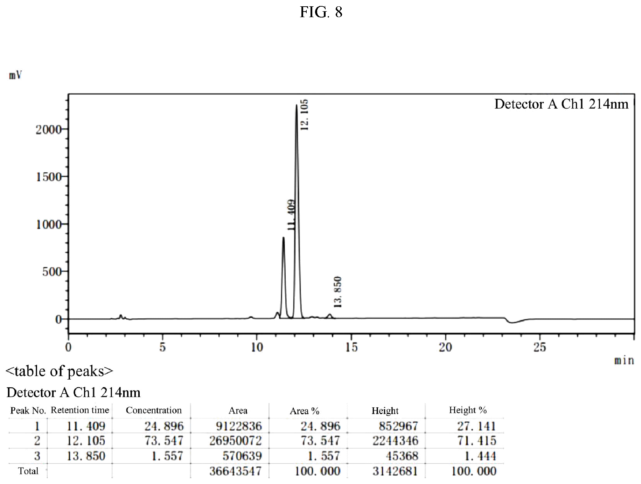

The method for detection of impurities in step 2 was the same as in Example 1. The results are shown in Table 2 and Figure 8.

### Comparative Example 5:

### Synthetic Scheme:

Compared to Example 1, step 1 was the same as in Example 1, and the difference was only in step 2. Specifically, in step 2: after purification by preparative liquid chromatography under acidic conditions (mobile phase: 0.1% TFA/water - 0.1% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated at low temperature (≤30°C) using a nanofiltration device, and lyophilized to obtain the target compound. For the specific conditions of the purification by preparative liquid chromatography, except that the mobile phase A was replaced with 0.1% TFA/water, the other specific conditions for the purification by preparative liquid chromatography were the same as in Example 1.

### Impurity detection method of step 2:

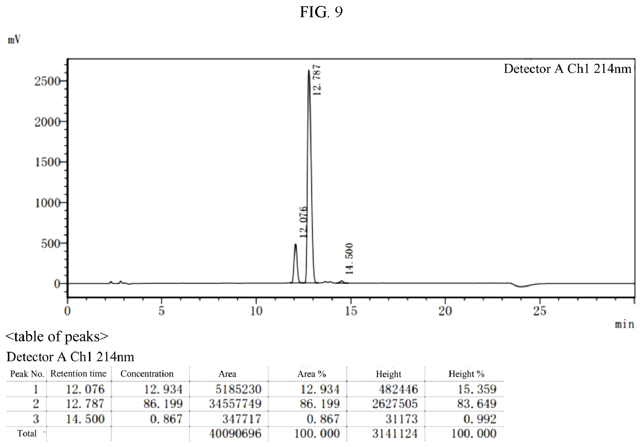

The method for detection of impurities in step 2 was the same as in Example 1. The results are shown in Table 2 and Figure 9.

### Comparative Example 6:

### Synthetic Scheme:

Compared to Example 1, step 1 was the same as in Example 1, and the difference was only in step 2. Specifically, in step 2: after purification by preparative liquid chromatography under acidic conditions (mobile phase: 0.1% TFA/water - 0.1% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated in a water bath at 35 °C under reduced pressure using an oil pump, and lyophilized to obtain the target compound. For the specific conditions of the purification by preparative liquid chromatography, except that the mobile phase A was replaced with 0.1% TFA/water, the other specific conditions for the purification by preparative liquid chromatography were the same as in Example 1.

### Impurity detection method of step 2:

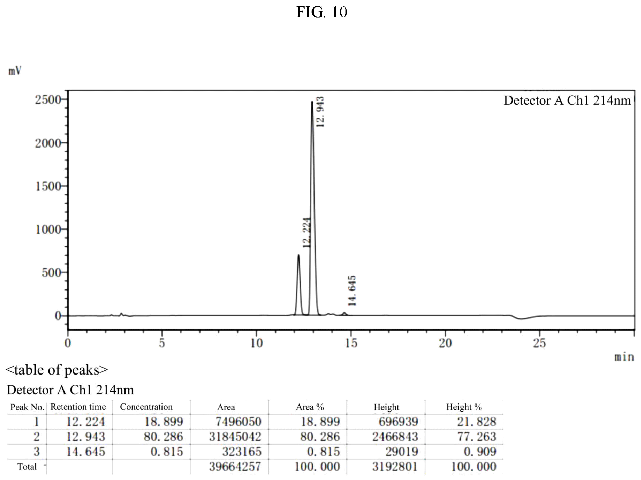

The method for detection of impurities in step 2 was the same as in Example 1. The results are shown in Table 2 and Figure 10.

### Comparative Example 7:

### Synthetic Scheme:

Compared to Example 1, step 1 was the same as in Example 1, and the difference was only in step 2. Specifically, in step 2: after purification by preparative liquid chromatography under acidic conditions (mobile phase: 0.2% TFA/water - 0.1% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated in a water bath at 35 °C under reduced pressure using an oil pump, and lyophilized to obtain the target compound. The specific conditions of the purification by preparative liquid chromatography were the same as in Example 1.

### Impurity detection method of step 2:

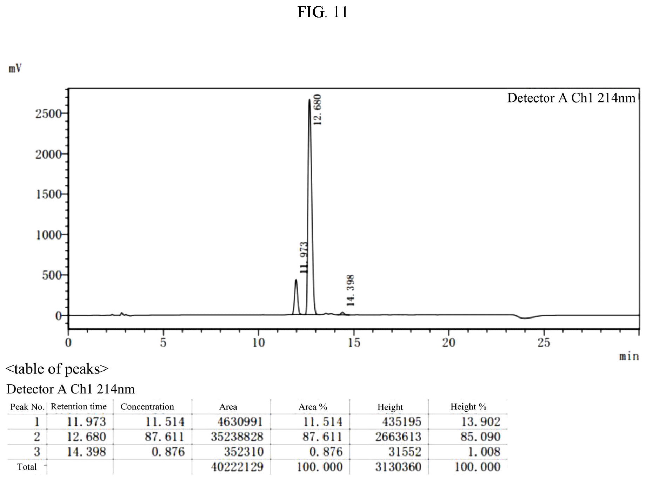

The method for detection of impurities in step 2 was the same as in Example 1. The results are shown in Table 2 and Figure 11 below.

### Comparative Example 8:

### Synthetic Scheme:

Compared to Example 1, step 1 was the same as in Example 1, and the difference was only in step 2. Specifically, in step 2: after purification by preparative liquid chromatography under acidic conditions (mobile phase: 0.2% TFA/water - 0.2% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated at low temperature (≤30°C) using a nanofiltration device, and lyophilized to obtain the target compound. For the specific conditions of the purification by preparative liquid chromatography, except that the mobile phase B was replaced with 0.2% TFA/acetonitrile, the other specific conditions for the purification by preparative liquid chromatography were the same as in Example 1.

### Impurity detection method of step 2:

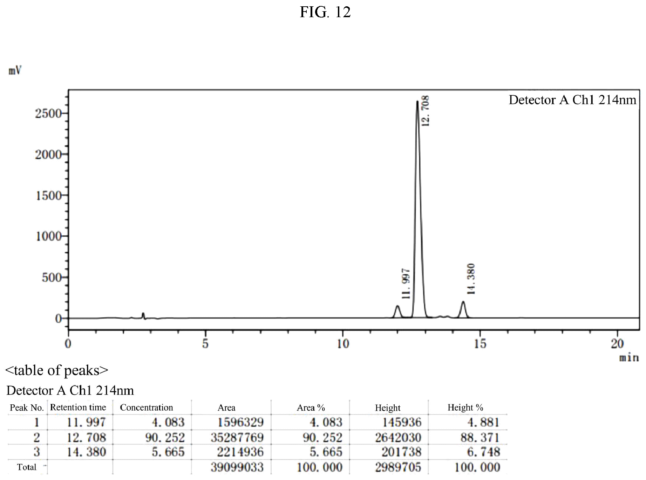

The method for detection of impurities in step 2 was the same as in Example 1. The results are shown in Table 2 and Figure 12.

### Comparative Example 9:

### Synthetic Scheme:

Compared to Example 1, step 1 was the same as in Example 1, and the difference was only in step 2. Specifically, in step 2: after purification by preparative liquid chromatography under acidic conditions (mobile phase: 0.2% TFA/water - 0.2% TFA/acetonitrile), a preparative solution (volume V) of the target compound was obtained. The organic solvent in the preparative solution was then extracted sequentially with ethyl acetate (0.75V)/n-hexane (0.25V) and dichloromethane (0.3V)/n-hexane (0.1V). The organic phases were discarded, and the aqueous phase was obtained. The aqueous phase was then concentrated in a water bath at 35 °C under reduced pressure using an oil pump, and lyophilized to obtain the target compound. For the specific conditions of the purification by preparative liquid chromatography, except that the mobile phase B was replaced with 0.2% TFA/acetonitrile, the other specific conditions for the purification by preparative liquid chromatography were the same as in Example 1.

### Impurity detection method of step 2:

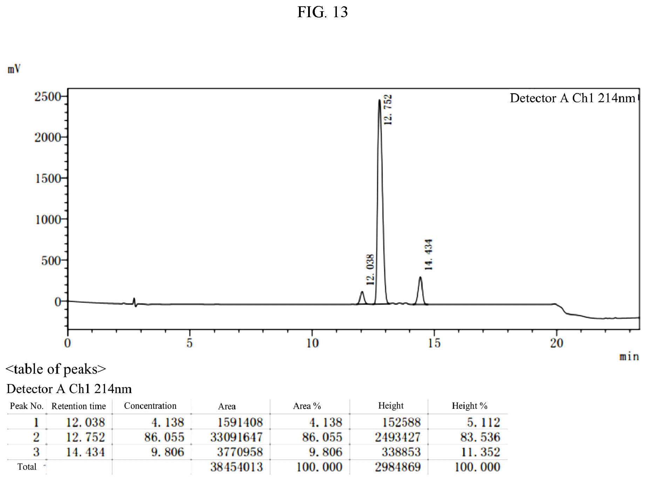

The method for detection of impurities in step 2 was the same as in Example 1. The results are shown in Table 2 and Figure 13.

**Table 2: Detection results of impurities**

| Mobile phase | Concentration operation | No. | Impurity status (HPLC peak area ratio) | | |
|---|---|---|---|---|---|
| | | | Ring-opening product | Target compound | Drug unit |
| Pure water-acetonitrile | / | Comparative Example 2 | 31.7% | 65.1% | 3.1% |
| 0.1%TFA/water-acetonitrile | Nanofiltration concentration (≤30°C) | Comparative Example 3 | 16.5% | 82.7% | 0.83% |
| | Concentration under reduced pressure at 35°C | Comparative Example 4 | 24.8% | 73.5% | 1.55% |
| 0.1%TFA/water-0.1%TFA/acetonitrile | Nanofiltration concentration (≤30°C) | Comparative Example 5 | 12.9% | 86.2% | 0.87% |
| | Concentration under reduced pressure at 35°C | Comparati ve Example 6 | 18.9% | 80.3% | 0.81% |
| 0.2%TFA/water-0.1%TFA/acetonitr ile | Nanofiltration concentration (≤30°C) | Example 1 | 2.1% | 96.9% | 1.01% |
| | Concentration under reduced pressure at 35°C | Comparative Example 7 | 11.5% | 87.6% | 0.88% |
| 0.2%TFA/water-0.2%TFA/acetonitrile | Nanofiltration concentration (≤30°C) | Comparative Example 8 | 4.0% | 90.3% | 5.7% |
| | Concentration under reduced pressure at 35°C | Comparative Example 9 | 4.1% | 86.1% | 9.8% |

The comparison of the results of Example 1 and Comparative Examples 2 to 9 clearly showed that, in the production process of the target compound, only the preparation method sought to be protected by the application could meet the product quality standards (ring-opening product must be controlled below 10%, and drug unit must be controlled below 5%), and even far exceed the quality standards (ring-opening product was as low as 3% or below, and drug unit was as low as 2% or below).

It should be understood that the present application is not limited to the specific methodologies, experimental procedures, or reagents described herein, as such aspects may vary. The discussions and examples are provided for illustrative purposes only and are not intended to limit the scope of the present application. The scope of the present application is defined by the claims.

## Claims

1. A method for preparing a compound represented by Formula A, comprising: 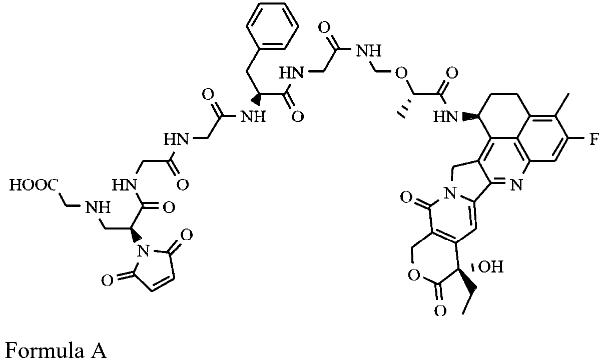
reacting a compound represented by Formula a with a Lewis acid to obtain a crude product; and
purifying the crude product to obtain the compound represented by Formula A; 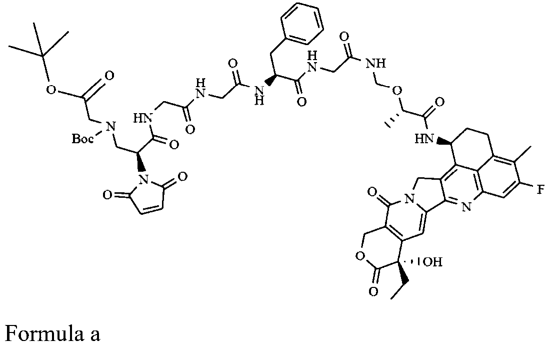
preferably, the Lewis acid is a bromide, TMSI, or TMSOTf;
more preferably, the Lewis acid is zinc bromide.

2. The method according to claim 1, wherein the purifying comprises the following steps:
(2-1) purifying the crude product by a preparative liquid chromatography to obtain a preparative solution;
(2-2) concentrating and drying the preparative solution to obtain the compound represented by Formula A;
optionally, the method further comprising an extraction step after obtaining the preparative solution in step (2-1) and before step (2-2);
optionally, after obtaining the preparative solution in step (2-1) and before step (2-2), the following step is further included:
(2-i) extracting the preparative solution with an organic solvent, discarding the organic phase, and obtaining the aqueous phase;
wherein in step (2-1), when purifying by the preparative liquid chromatography, the mobile phase A comprises TFA/water, and the mobile phase B comprises TFA/acetonitrile;
wherein in step (2-2), the concentrating comprises nanofiltration concentrating.

3. The method according to claim 2, wherein the method further comprises one or more technical features selected from the group consisting of the following (i) to (iii):
(i) in step (2-1), gradient elution is used when purifying by the preparative liquid chromatography;
the gradient conditions are:
| Time/min | Mobile phase A/% | Mobile phase B/% |
|---|---|---|
| 0~5 | 90~75 | 10~25 |
| 5~32 | 75~60 | 25~40 |
| 32~39 | 10~0 | 90~100 |
| 39~50 | 90~75 | 10~25 |
(ii) in step (2-1), when purifying by the preparative liquid chromatography, the detection wavelength is 200-365 nm;
(iii) in step (2-2), the nanofiltration is performed under conditions with a temperature of ≤30°C;
preferably, the nanofiltration is achieved using a nanofiltration device.

4. The method according to any one of claims 2 to 3, wherein the method further comprises one or more technical features selected from the group consisting of (i) to (ii):
(i) in step (2-2), the drying is lyophilizing;
(ii) in step (2-i), the organic solvent is mixed solvent 1 and/or mixed solvent 2, wherein the mixed solvent 1 comprises ethyl acetate and n-hexane, and the mixed solvent 2 comprises dichloromethane and n-hexane;
preferably, step (2-i) is performed by sequentially extracting the preparative solution with the mixed solvent 1 and the mixed solvent 2, discarding the organic phases, and obtaining the aqueous phase;
preferably, the volume ratio of the mixed solvent 1 to the preparative solution is (1-5):1, more preferably 1:1;
preferably, the volume ratio of the preparative solution to the mixed solvent 2 is (1-5):1, more preferably 5:2;
preferably, in the mixed solvent 1, the volume ratio of ethyl acetate to n-hexane is (1-5):1, more preferably 3:1;
preferably, in the mixed solvent 2, the volume ratio of dichloromethane to n-hexane is (1-5):1, more preferably 3:1.

5. The method according to any one of claims 1 to 4, wherein the method further comprises one or more technical features selected from the group consisting of following (i) to (iv):
(i) the molar ratio of the Lewis acid to the compound represented by Formula a is not less than 10:1;
(ii) the reacting is carried out in the presence of a solvent, and the solvent is selected from the group consisting of nitroalkanes (e.g., nitromethane, nitroethane), haloalkanes (e.g., dichloromethane, dichloroethane, chloroform), water, ketones (e.g., acetone, butanone, etc.), toluenes (e.g., toluene, trifluorotoluene), esters (e.g., ethyl acetate, dimethyl carbonate), acetonitrile, and mixed solvents obtained by mixing two or more of the above solvents in any proportion, preferably selected from the group consisting of nitromethane, dimethyl carbonate, dichloromethane, mixed solutions of dichloromethane/ketones, mixed solutions of dichloromethane/water, and mixed solutions of dichloromethane/esters;
(iii) the reacting is carried out at a temperature of 20-110°C;
(iv) the reacting is carried out for a duration of 20 minutes to 24 hours.

6. The method according to any one of claims 1 to 5, wherein after reacting the compound represented by Formula a with the Lewis acid and before purifying, the method further comprises a post-treatment step;
preferably, the post-treatment comprises (i) or (ii):
(i) concentrating the obtained reactant under reduced pressure to obtain the crude product;
(ii) crystallizing, filtering, and drying the obtained reactant to obtain the crude product;
preferably, the concentrating under reduced pressure is carried out at a temperature of 30-55°C.

7. The method according to any one of claims 1 to 6, wherein the compound represented by Formula a is prepared by the following methods:
(3-1-1) reacting a compound represented by Formula b with a base to obtain a compound 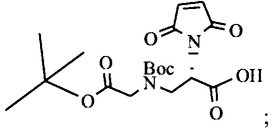 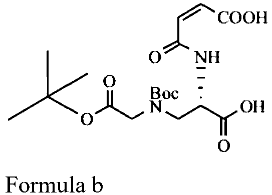
(3-1-2) reacting the compound 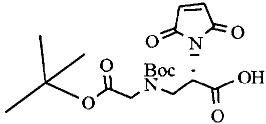 with a compound 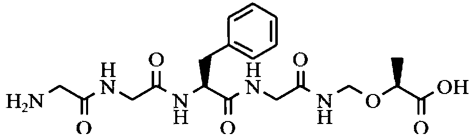 to obtain a compound 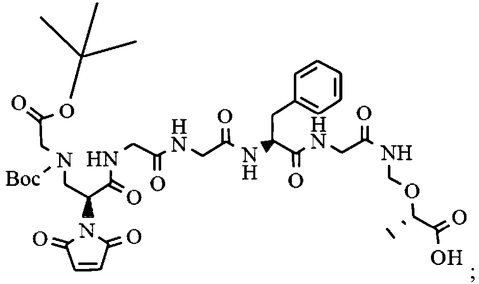
(3-1-3) reacting the compound 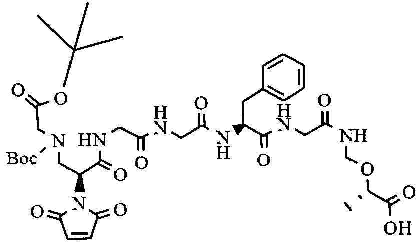 with exatecan mesylate to obtain the compound represented by Formula a;
alternatively, the compound represented by Formula a is prepared by the following methods:
(3-2-1) reacting a compound represented by Formula b with a base to obtain a compound 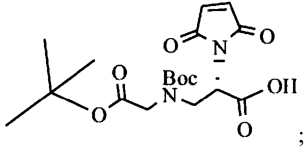 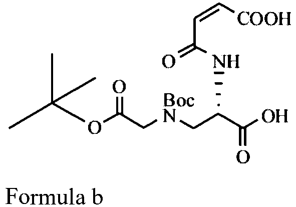
(3-2-2) reacting the compound 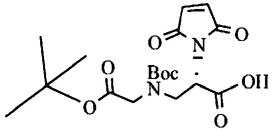 with pentafluorophenol and DCC to obtain a compound 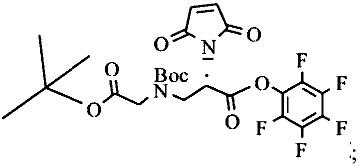
(3-2-3) reacting the compound 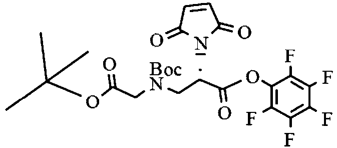 with a compound 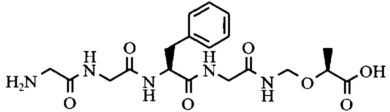 to obtain a compound 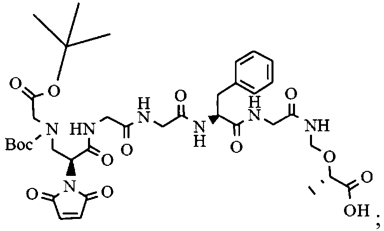
(3-2-4) reacting the compound 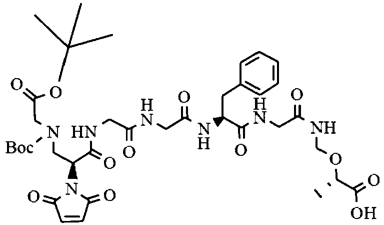 with exatecan mesylate to obtain the compound represented by Formula a.

8. The method according to claim 7, wherein the compound represented by Formula b is prepared by the following methods:
(4-1) reacting a compound 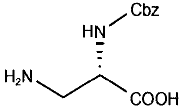 with thionyl chloride and benzyl alcohol to obtain a compound 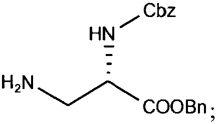
(4-2) reacting the compound 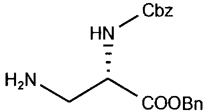 with a compound 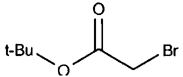 to obtain a compound 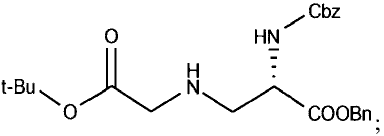
(4-3) reacting the compound 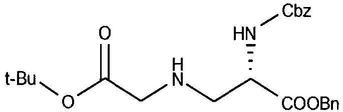 with (Boc)₂O to obtain a compound 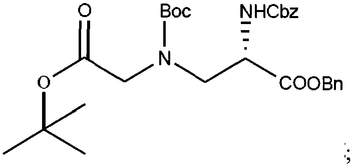
(4-4) reacting the compound 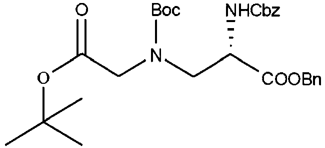 with hydrogen gas in the presence of Pd/C to obtain a compound 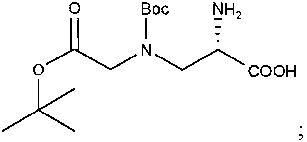
(4-5) reacting the compound 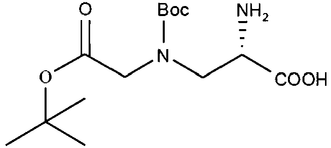 with maleic anhydride to obtain the compound represented by Formula b.

9. The method according to claim 8, wherein step (4-1) is carried out by the following steps:
(4-1-1) adding thionyl chloride dropwise to benzyl alcohol, followed by adding the compound 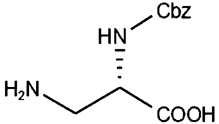 to react;
(4-1-2) adding isopropyl ether to the reactant obtained in step (4-1-1) to induce crystallization, filtering, and drying the filter cake to obtain the compound

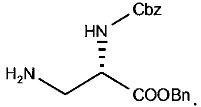

10. The method according to claim 8, wherein step (4-4) is carried out by the following steps:
(4-4-1) reacting the compound 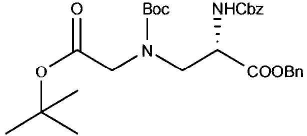 with hydrogen gas in the presence of Pd/C;
(4-4-2) filtering the reactant obtained in step (4-4-1), and concentrating the filtrate under reduced pressure to obtain a crude product;
(4-4-3) recrystallizing the crude product with isopropanol/water to obtain a crystal, which is the compound 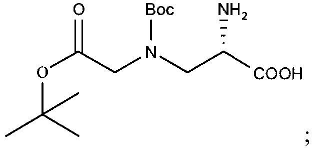
preferably, when recrystallizing, the volume ratio of isopropanol to water is (1-5):1, more preferably 3:1.

11. The method according to claim 8, wherein step (4-5) is carried out by the following steps:
(4-5-1) reacting the compound 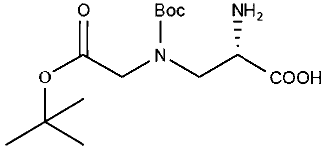 with maleic anhydride;
(4-5-2) adding n-hexane to the reactant obtained in step (4-5-1) to induce crystallization, filtering, and drying the filter cake to obtain the compound represented by Formula b.
